# EUROPEAN PATENT APPLICATION

(11) **EP 3 006 071 A1**
(43) Date of publication of application: **13.04.2016**
(21) Application number: 14382383.9
(22) Date of filing: 07.10.2014
(51) Int. Cl.: A61M 16/00, A61M 16/06

(54) **Demand oxygen delivery system**

(71) Applicant: Hersill, S.L., 28935 Mostoles Madrid (ES)
(72) Inventor: Martínez Jordán, Carlos, 28935 Madrid (ES)
(74) Representative: Stiebe, Lars Magnus

(57) **Abstract**

The present invention refers to a device (10) for demand oxygen delivery to a patient, which comprises means for delivering an amount of oxygen (30) to the patient, characterised in that the device further comprises:
- means for identifying an absence of the patient's inspiration during a pre-established or calculated period of time (206, 220, 222);
- means for delivering a sensorial warning by touch (221, 223) upon identifying such absence; and/or,
- means for adjusting the amount of oxygen delivered to the patient according to a patient's respiratory frequency (210-218).

The sensorial warning by touch may comprise a train of oxygen pulses with a predefined duration and rate, delivered to the patient's nose via a nasal cannula (30).

## Description

### TECHNICAL FIELD

The invention relates to the field of medical devices, more particularly to supplemental medicinal gas delivering devices designed to supply to a patient effective amounts of medicinal gases such as oxygen.

### STATE OF THE ART

Devices for delivering oxygen to a patient using an intermittent pulsed flow are commonly used in long-term home oxygen therapy for patients with chronic obstructive pulmonary disease. These devices are used together with a nasal cannula.

There are several types of devices for delivering oxygen to a patient.

There is a first type of devices for delivering oxygen which supply oxygen to the patient in a continuous manner. This sort of device consumes an important quantity of oxygen, which can imply a reduction in the autonomy of the patient.

A second group of devices, known as demand oxygen delivery systems (DODS), supply oxygen to the patient only during a fixed period of time.

The DODS is able to detect the beginning of the inspiration thanks to the fact that the patient generates a vacuum that is transmitted to the device through the nasal cannula. After this detection the system supplies oxygen to the patient, through the nasal cannula, during a fixed period of time. In this way, the supply of oxygen from the DODS to the patient is synchronized with his breathing.

There are two types of DODS: purely pneumatic DODS and electronic DODS.

The advantage of the purely pneumatic DODS over the electronic DODS is that they do not need batteries or electric supply for their operation. Their disadvantage is that they require an important level of vacuum to enable the trigger that launches the oxygen flow, because this vacuum has to create the force needed to operate a mechanical device that starts a pneumatic timer; this means that this level of vacuum needs a time to be reached (delay on the inspiration) and even not all the patients can reach the required level.

The advantage of the electronic DODS over the pneumatic ones is that the vacuum is detected by a device, such as a pressure switch or a pressure sensor, and the oxygen flow is launched by a different device (electro-valve). This way, the trigger can be detected at the beginning of the inspiration period, and consequently the amount of oxygen needed to achieve the same physiological effect is smaller. The clinical explanation is that only the oxygen inhaled during the initial period of the inspiration reaches the alveoli and is absorbed by the blood; so that the faster the oxygen is launched the lower amount of oxygen is needed.

The main problem with any DODS is that its effectiveness depends heavily on:
- the patient inspiration being by the nasal way;
- the nasal cannula being correctly placed;
- the patient produces enough vacuum to trigger the device:
- using an appropriate type of nasal cannula.

So the DODS becomes ineffective if it is unable to detect the inspiration effort of the patient. This happens when the patient breaths through the mouth; the patient breathes though the nose but he is not capable of triggering the device because he is not doing sufficient inspiring effort, or because the nasal cannula is not properly placed, or the orifice of the nasal cannula is too narrow.

Even if the importance of these facts is explained to the patient, after a while the patient ceases to pay attention and the system will be less effective.

An additional problem is that the system becomes less effective precisely when the patient needs more oxygen, since when an effort is made the patient tends to breathe through the mouth.

### DESCRIPTION OF THE INVENTION

It is an object of the present invention to provide a device for demand oxygen delivery to a patient that warns the patient when his/her inspiration is not detected. In order to do so, the device of the present invention produces a sensorial warning by touch to the patient when the device detects that the patient is not inspiring through the nose during a pre-established or calculated period of time.

Accordingly, a first aspect of the invention refers to a device for demand oxygen delivery to a patient which comprises means for delivering an amount of oxygen to the patient, the device further comprising:
- means for identifying an absence of the patient's inspiration during a pre-established or calculated period of time;
- means for delivering a sensorial warning by touch upon identifying such absence.

That is, the device of the present invention can operate to identify the absence of the patient's breath: with a pre-established period of time and with a calculated period of time.

The sensorial warning by touch preferably comprises a train of oxygen pulses. The number of pulses and their duration can vary. These oxygen pulses provide a sensorial warning that can be sensed in the skin or in the nasal mucosa, and that could even be audible.

According to a preferred embodiment, the device further comprises a means for connecting a nasal cannula, and the means for delivering such sensorial warning by touch to the patient is arranged to deliver the sensorial warning by touch to the patient's nose via the nasal cannula. Consequently, the same nasal cannula is used for three purposes: to detect the vacuum generated in the patient, to supply oxygen to the patient after the inspiration detection, and to deliver the sensorial warning to the patient.

In an advantageous embodiment of the device of the invention, if after the first train of oxygen pulses the device detects that the patient is still not inspiring during another pre-established or calculated period of time, the device can provide a second train of oxygen pulses, which can be different in time, duration and number of pulses than the first train.

The device may further comprise means for calculating a respiratory frequency of the patient, and the calculated period of time can be then calculated as a function of the patient's respiratory frequency. The respiratory frequency of the patient can be continuously stored in a memory of the device for future analyses.

The amount of oxygen delivered to the patient by the device of the present invention can be adjusted depending on the patient's respiratory frequency. For achieving this aim, the device preferably further comprises means for modifying the amount of oxygen delivered to the patient (oxygen bolus) in accordance with the difference between the current respiratory frequency and the basal respiratory frequency of the patient (the basal respiratory frequency is the breathing frequency in rest conditions).

The patient's basal respiratory frequency is a parameter that can be introduced by a user in the device. Or it is also possible that the device further comprises means for calculating the basal respiratory frequency of the patient.

The basal respiratory frequency of the patient can be calculated by analysing the breathing patterns of the patient from a historical record stored in an internal memory provided in the device, by identifying the rest periods and the activity periods within the breathing patterns; the basal respiratory frequency of the patient is the average rest breathing frequency.

In another particular embodiment, the device further comprises means for adjusting the amount of oxygen delivered to the patient according to a clinical prescription of the patient.

In a particular embodiment, the device of the invention further comprises means for connecting a nasal cannula and:
- a pressure micro-sensor for providing a pressure measurement inside the nasal cannula;
- processing means for processing the pressure measurement to decide whether an inspiration has taken place, and upon such decision, detecting if the pressure measurement is below a pre-established vacuum threshold value; and,
- trigger means for triggering the delivery of the amount of oxygen to the patient upon such detection.

The device may further comprise means for enabling a user to adjust the vacuum threshold value from a keyboard interface, in terms of the measured pressure in the nasal cannula, which is preferably displayed in a display provided in the device.

According to another preferred embodiment, the device further comprises trigger means for triggering the delivery of the amount of oxygen to the patient through the nasal cannula, upon detecting that the pressure inside the nasal cannula is below the pre-established vacuum threshold value. The pre-established vacuum threshold value can be adjustable by a user, for example, by means of a keyboard.

By means of the device of the present invention, thanks to the sensorial warning, the patient is warned, trained and educated on breathing correctly in the long term, which leads to an efficient use of the device. Breathing correctly means to inspire by the nasal way and maintaining the nasal cannula correctly placed.

In addition to the sensorial warning capability of the device of the present invention, the device may further include the capability of adjusting the vacuum threshold value that triggers the delivery of the amount of oxygen (oxygen bolus) to the patient. Therefore, by means of this device it is possible to set the trigger level according to the patient's needs, and avoid false triggers.

It is another object of the present invention to provide a device that is able to modify the amount of oxygen delivered to the patient (oxygen bolus) according to the patient's respiratory frequency, which capability is called self-adjustable bolus.

For this purpose, the device preferably further comprises means for modifying the amount of oxygen delivered to the patient (oxygen bolus) in accordance with the difference between the current respiratory frequency and the basal respiratory frequency of the patient.

That is, in addition to the sensorial warning and the adjustable trigger level capabilities, the device can further include the self-adjustable bolus capability, that is, the capability of analysing the breathing pattern of the patient to adjust the amount of oxygen to be delivered to the patient. During the patient's day to day life, the patient may change their oxygen needs. These different oxygen needs, depending on the different activity situation of the patient, results in changes on the breathing pattern of the patient, that the device analyses to adjust the amount or quantity of oxygen (bolus) delivered to the patient after every trigger.

The device of the invention as described hereinbefore is preferably portable.

The different aspects and embodiments of the invention defined in the foregoing can be combined with one another, as long as they are compatible with each other.

Additional advantages and features of the invention will become apparent from the detailed description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a view of a device for delivering oxygen according to the invention.
Figure 2 schematically shows the main hardware elements of the device.
Figure 3 shows a flowchart of the device of the invention, showing the operation of the sensorial warning and self-adjustable bolus capabilities.
Figure 4 shows three possible examples of the train pulses generated by the sensorial warning capability of the device.

### DESCRIPTION OF A PREFERRED EMBODIMENT

The following description is not to be taken in a limiting sense but is given solely for the purpose of describing the broad principles of the invention. Next embodiments of the invention will be described by way of example, with reference to the above-mentioned drawings showing elements and results according to the invention.

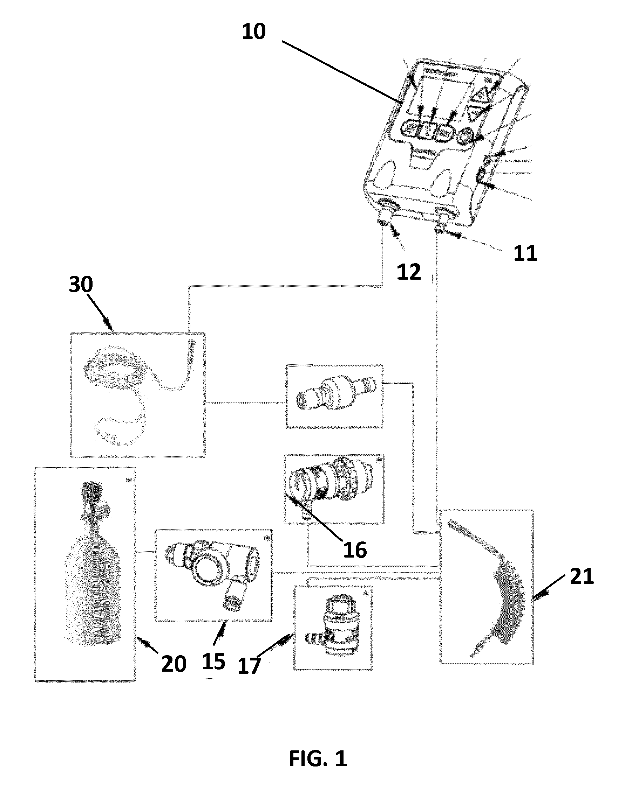

As shown in Figure 1, the device 10 of the present invention is a portable device intended to provide an additional oxygen supply to patients with difficulty to obtain it, naturally, from the breathed ambient air.

The device 10 has an inlet 11 for connecting an oxygen source 20 via a hose 21, and an outlet 12 for connecting a nasal cannula 30 that must be placed in the patient head with its orifices in the nose.

The oxygen delivery takes place when the patient initiates each inspiration, and its physiological effect is equivalent to that produced by means of a continuous-flow oxygen-therapy device, but with much lower oxygen consumption.

The device 10 of the invention immediately reacts to the patient's inspiration effort, and triggers the delivery of a pulsed volume of oxygen of fixed flow and variable time (bolus) through the nasal cannula 30. The volume of the supplied oxygen in every pulse (bolus) is measured in mL and it is possible to determine, thanks to experiments with patients, the equivalence between bolus (mL) and continuous flow (mL/min). In consequence, if the user sets in the unit a flow of x L/min, the device delivers, after each inspiration, an oxygen pulse (bolus) whose physiological effect is similar to the produced by a continuous flow of x L/min.

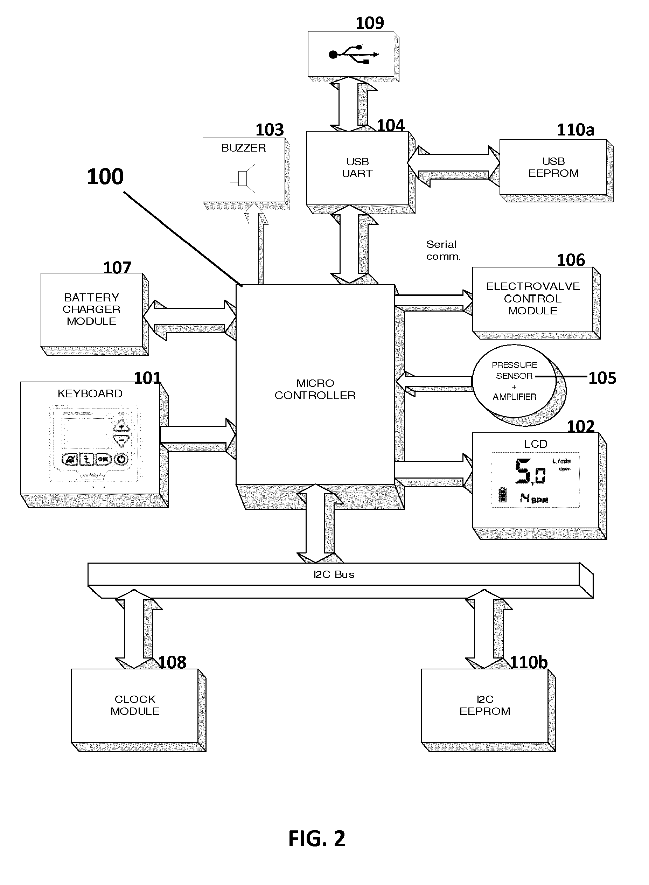

For the operation of the device 10, it is very important to detect the beginning of the patient's inspiration as soon as possible. For this purpose the device 10 comprises a pressure micro-sensor 105 (see Fig. 2). The pressure micro-sensor monitors the pressure through the nasal cannula 30. This measure of pressure requires great sensibility since the nasal cannula does not reflect the actual pressure of the airway because the nasal cannula is not perfectly sealed to the device. To avoid false triggers, the pressure measurements from the micro-sensor are processed by an adaptive selective algorithm to take the decision whether the inspiration has taken place or not. This algorithm filters the pressure measurements in order to reject false spurious values that can be generated by speech, coughing and/or irregular breathing. The filter is based in rejecting those pressure measurements that are not confirmed for not being a valid tendency in view of the previous and following measurements. If this processing confirms that the pressure measurement is valid and that it is below a pre-established vacuum threshold value, the device 10 triggers the delivery of the oxygen bolus.

The device 10 includes the possibility of configuring the vacuum threshold value by adjusting the trigger level from -1 mmH₂O to -9,5 mmH₂O, so as to optimise the device 10 for each patient, allowing its adaptation to the specific respiratory needs of patients who would not be able to use other saving systems: either their breathing is too strong thereby causing false triggers, or their breathing is too weak and not sufficient for activating the trigger.

Figure 2 shows the main hardware modules that form the device of the present invention. The device 10 includes a main process module (microcontroller 100) that has a primary user interface with inputs (a keyboard 101) and outputs (an LCD 102 and a Buzzer 103), and an advanced communications interface (USB UART 104). The patient's pressure is measured by a micro-sensor module (pressure micro-sensor 105 + amplifier). The pressure measurements from the micro-sensor are processed by the adaptive selective algorithm of the main process module (microcontroller 100), which decides whether the inspiration has taken place or not, and the measured pressure is compared with the pre-established vacuum threshold value. If the decision is that a bolus has to be triggered (the patient has inspired and the pressure measurement is below the pre-established vacuum threshold value), the main process module sends an order to an output pneumatic module (electrovalve control module 106) to open the valve. The device 10 further includes other standard components of a portable electronic device, such as a battery charger module 107, a clock module 108 for operation of the microcontroller 100 and management of data and registers in memory modules 110a and 100b, and a USB port 109 for communication purposes.

The device of the invention can be used in homecare oxygen-therapy and in hospital or medical installations, because it can be supplied from a high pressure cylinder (20, 15 and 17, in Figure 1), from a liquid oxygen (LOX) container and from a low pressure hospital oxygen pipeline (16 in Figure 1).

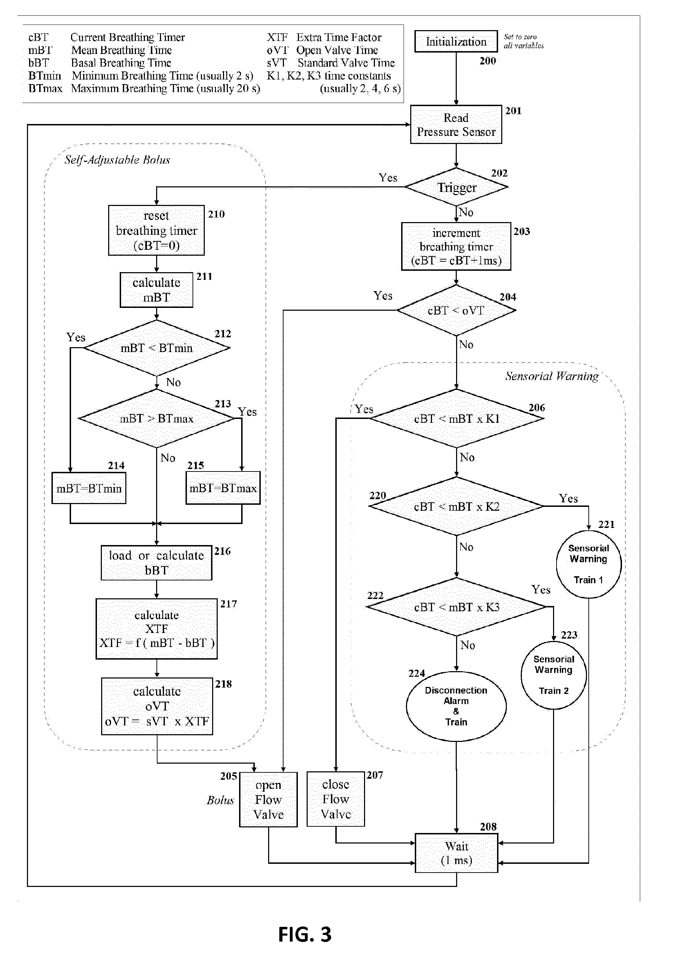

Figure 3 shows a flowchart of the preferred embodiment of the device of the invention, showing the operation of the sensorial warning and self-adjustable bolus capabilities.

First, the device is initialised (200), so all variables are set to zero. Then, the pressure in the micro-sensor 105 is read (201), and the main process module processes and filters such pressure measurement -to reject false values- to decide whether a bolus has to be triggered (202). If it is decided that a bolus should be triggered, the device opens the electrovalve 106 (205), but it previously activates its self-adjustable bolus capability which provides the device with the capability of modifying the amount of oxygen delivered to the patient (oxygen bolus), by means of calculating the time that the electrovalve must be opened (open valve time, oVT).

When the self-adjustable bolus capability is activated, a current breathing timer, cBT, is initiated (210) which measures the time in the current breathing cycle.

The mean breathing time, mBT, of the patient (211) is calculated as the average breathing time in the previous breathing cycles, using the patient's respiratory frequency history uploaded in the EEPROM memories 110a, 110b.

In order to avoid erroneous values of the mean breathing time mBT, a minimum breathing time BTmin and a maximum breathing time BTmax, of for example 2 and 20 seconds (which correspond to breathing frequencies of 30 and 3, respectively), are established. So it is checked (212) if the mean breathing time mBT is below the minimum breathing time, BTmin; if so, the mean breathing time mBT is equalled to the minimum breathing time BTmin (214). If not, it is checked (213) if the mean breathing time mBT is above the maximum breathing time BTmax. If so, the mean breathing time mBT is equalled to the maximum breathing time BTmax (215).

The basal breathing time, bBT (which is the breathing time of the patient in rest conditions), is then loaded in the device. Or it can be calculated by the device using the patient's respiratory frequency history uploaded in the EEPROM memories 110a, 110b.

Then the difference between the mean breathing time mBT and the basal breathing time bBT is calculated to determine whether the patient is doing some extra effort (the bigger is the difference the bigger is the oxygen need with regards to the rest conditions of the patient). A parameter called 'extra time factor', XTF, is then calculated (217) as a function of that difference between the mean breathing time mBT and the basal breathing time bBT.

Finally, the target of the self-adjustable bolus capability is achieved by calculating the variable 'open valve time', oVT, that defines how long the electrovalve 106 will be opened over the standard valve time, sVT (which is the standard pre-set time during which the valve is open in rest conditions). The open valve time, oVT, is calculated (218) as the standard valve time, sVT, times the extra time factor, XTF. Then the electrovalve 106 will be opened (205) and will remain open during the time set in 'open valve time' oVT (204), delivering an amount of oxygen to the patient according to the patient's respiratory frequency. For instance, XTF can be 1.05, so that the valve is open during 5% more time than in the standard case to cover the extra effort of the patient.

If in 202 it is decided that a bolus should be not triggered, the current breathing timer cBT is incremented (203) in each loop (208) in, for example, 1 ms. The current breathing timer cBT is then compared with the open valve time oVT (204), so as to decide whether to maintain the electrovalve open (205) or whether to go the next decision steps, grouped in what is called the sensorial warning capability. The electrovalve will be kept open (205) as long as the current breathing timer cBT is shorter than the open valve time oVT. The first time the current breathing timer cBT exceeds the open valve time oVT the electrovalve will be closed (207).

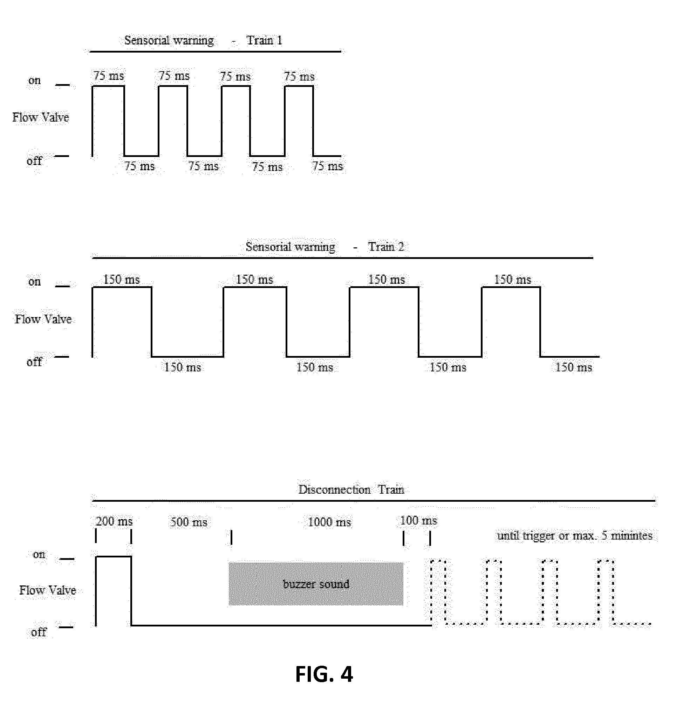

The sensorial warning capability contains three time comparators (206, 220, 222), focused to decide if it is necessary to generate a first sensorial warning (221), a second sensorial warning (223) or a disconnection alarm (224). In order to do so, it is checked if the current breathing timer cBT is shorter or longer than K1, K2 and K3 times the mean breathing time mBT (206, 220, 222). If the current breathing timer cBT is longer than K1 times the mean breathing time mBT, a first train of oxygen pulses 'Train 1' (shown in Fig. 4) is sent to the patient (221) via the nasal cannula, which serves as a sensorial warning to the patient that he/she is not breathing properly. If the current breathing timer cBT is longer than K2 times the mean breathing time mBT, a second train of oxygen pulses 'Train 2' (shown in Fig. 4) is sent to the patient (223) via the nasal cannula, which serves as a further sensorial warning to the patient that he/she is not breathing properly. The 'Train 2' is different to 'Train 1' in order to cause a different feeling in the patient ('Train 2' is intended to be more disturbing than 'Train 1'). If the current breathing timer cBT is longer than K3 times the mean breathing time mBT, the device considers that the patient is not present or it is in apnea, both situations are sufficient cause to generate an audible alarm (buzzer 103 sound) combined with a longer train of oxygen pulses, focused to intend to maintain the patient oxygenated for a time. This third event is called 'Disconnection alarm & train' (224) (shown in Fig. 4).

As shown in Figure 4, the oxygen pulses in the second train of oxygen pulses (150 ms) are longer than the oxygen pulses in the first train (75 ms) which, as indicated previously, is intended to provide a more disturbing sensation to the patient. In both cases the train of oxygen pulses contains four pulses, but they could have different number of pulses. The 'Disconnection train' combines a first short oxygen pulse (of, for example, 200 ms), with a longer audible sound in the buzzer 103; this is repeated during a maximum configurable time (for example 5 minutes) to avoid that the oxygen cylinder is completely emptied. This way, the disconnection alarm is no only a sensorial warning for the patient, but also provides an audible alarm for the medical staff (the lack of breathing can be caused by an apnea).

In this text, the term "comprises" and its derivations (such as "comprising", etc.) should not be understood in an excluding sense, that is, these terms should not be interpreted as excluding the possibility that what is described and defined may include further elements, steps, etc.

In the context of the present invention, the term "approximately" and its derivatives should be understood as indicating values very near to those which they accompany. That is to say, a deviation within reasonable limits from an exact value should be accepted, because a skilled person will understand that such a deviation from the indicated values is inevitable due to measurement inaccuracies, etc. The same applies to the terms "about" and "around" and "substantially".

## Claims

1. Device (10) for demand oxygen delivery to a patient, which comprises means for delivering an amount of oxygen (30) to the patient, **characterised in that** the device further comprises:
- means for identifying an absence of the patient's inspiration during a pre-established or calculated period of time (206, 220, 222);
- means for delivering a sensorial warning by touch (221, 223) upon identifying such absence.

2. Device (10) according to claim 1, wherein the sensorial warning by touch comprises a train of oxygen pulses with a predefined duration and rate.

3. Device (10) according to any of claims 1-2, wherein the device further comprises means for connecting a nasal cannula (30), and wherein the means for delivering such sensorial warning by touch to the patient is arranged to deliver the sensorial warning by touch to the patient's nose via the nasal cannula (30).

4. Device (10) according to any of claims 1-3, which further comprises means for calculating a respiratory frequency of the patient, and wherein the calculated period of time is a function of the patient's respiratory frequency.

5. Device according to any of claims 1-4, wherein the amount of oxygen delivered to the patient is adjusted depending on a patient's respiratory frequency (210-218).

6. Device according to any of claims 1-5, which comprises means for modifying the amount of oxygen delivered to the patient as a function of the difference between the respiratory frequency of the patient and a basal respiratory frequency of the patient.

7. Device according to claim 6, wherein the basal respiratory frequency is calculated by analysing breathing patterns of the patient recorded in a memory of the device.

8. Device according to claim 6, wherein the patient's basal respiratory frequency is a parameter that can be introduced by a user in the device.

9. Device according to any of claims 1-8, which further comprises means for connecting a nasal cannula (30), and:
- a pressure micro-sensor (105) for measuring a pressure inside the nasal cannula (30);
- processing means for processing the pressure measurement to decide whether an inspiration has taken place, and upon such decision, detecting if the pressure measurement is below a pre-established vacuum threshold value; and,
- trigger means for triggering the delivery of the amount of oxygen to the patient upon such detection.

10. Device (10) according to claim 9, which further comprises:
- means for enabling a user to adjust the vacuum threshold value from a keyboard interface (101), in terms of the measured pressure in the nasal cannula (30).

11. Device (10) for demand oxygen delivery to a patient, which comprises means for delivering an amount of oxygen (30) to the patient, **characterised in that** the device further comprises means for adjusting the amount of oxygen delivered to the patient according to a patient's respiratory frequency (210-218).

12. Device (10) according to claim 11, which further comprises which comprises means for modifying the amount of oxygen delivered to the patient as a function of the difference between a measured respiratory frequency of the patient and a basal respiratory frequency of the patient.

13. Device (10) according to any of claims 11-12, further comprising means for connecting a nasal cannula (30) for delivering an amount of oxygen to the patient, and:
- a pressure sensor (105) for measuring the pressure inside the nasal cannula (30);
- processing means for processing the pressure measurement to decide whether an inspiration has taken place, and upon such decision, detecting if the pressure measurement is below a pre-established vacuum threshold value; and,
- trigger means for triggering the delivery of the amount of oxygen to the patient upon such detection.

14. Device (10) for demand oxygen delivery to a patient, **characterised in that** the device comprises:
- means for identifying an absence of the patient's inspiration during a pre-established or calculated period of time;
- means for delivering a train of oxygen pulses with a predefined duration and rate upon identifying such absence.

15. Device according to any of claims 1-14, wherein the device is portable.
